Europäisches Patentamt

European Patent Office

Office européen des brevets

(11) Veröffentlichungsnummer: **0 523 376 A1**

# (12) EUROPÄISCHE PATENTANMELDUNG

(21) Anmeldenummer: **92109977.6**

(22) Anmeldetag: **13.06.92**

(51) Int. Cl.5: **A61K 47/14**

(30) Priorität: **20.06.91 DE 4120311**

(43) Veröffentlichungstag der Anmeldung:
**20.01.93 Patentblatt 93/03**

(84) Benannte Vertragsstaaten:
**AT BE CH DE DK ES FR GB GR IT LI LU NL PT SE**

(71) Anmelder: **Kali-Chemie Pharma GmbH**
**Hans-Böckler-Allee 20 Postfach 220**
**W-3000 Hannover 1(DE)**

(72) Erfinder: **Bonnacker, Ingo**
**Sedanstrasse 6**
**W-3250 Haneln(DE)**
Erfinder: **Klimek, Rainhardt**
**Thönser Trift 2**
**W-3006 Burgwedel 5(DE)**
Erfinder: **Stemmle, Berthold**
**Velperstrasse 14**
**W-3167 Burgdorf(DE)**
Erfinder: **Feicho, Lutz-Eitel**
**Kantstrasse 20**
**W-3167 Burgdorf(DE)**

(74) Vertreter: **Lauer, Dieter, Dr.**
**c/o Solvay Deutschland GmbH, Postfach 220**
**W-3000 Hannover(DE)**

(54) **Konservierte pharmazeutische Zubereitungen.**

(57) Die Erfindung bezieht sich auf eine Konservierungsmittelkombination aus p-Hydroxybenzoesäureethylester, p-Hydroxybenzoesäurepropylester und p-Hydroxybenzoesäurebutylester, die aufgrund ihrer Zusammensetzung eine sehr wirksame Konservierung von wäßrigen alkalischen Zubereitungen pharmazeutischer Wirkstoffe ermöglicht. In den PHB-Esterkombinationen kann jeder der drei genannten Ester durch sein Natriumsalz teilweise oder vollständig ersetzt sein.

EP 0 523 376 A1

Die vorliegende Erfindung bezieht sich auf konservierte wäßrige alkalische pharmazeutische Zubereitungen von Wirkstoffen, die eine Kombination aus sogenannten PHB-Estern, d.h., den Estern der p-Hydroxybenzoesäure mit niederen Alkoholen als Konservierungsmittel enthalten.

Wäßrigen Arzneimittelpräparaten, welche in zur Mehrfachdosierung geeigneten Gefäßen vertrieben werden, werden üblicherweise Konservierungsstoffe beigegeben, um Kontaminationen durch Mikroorganismen während der Lagerung und des Gebrauchs auszuschließen. Einige PHB-Ester, z.B. p-Hydroxybenzoesäuremethylester oder p-Hydroxybenzoesäurepropylester, sind als Konservierungsmittel für wäßrige Arzneimittelzubereitungen bekannt und werden häufig als Zweierkombination eingesetzt. Nach H.P. Fiedler, Lexikon der Hilfsstoffe für Pharmazie, Kosmetik und angrenzende Gebiete, Editio Cantor KG, Aulendorf i. Württ., 3. Auflage, 1989 wird zur Konservierung von insbesondere Arzneimitteln ein Konservierungsmittel, das aus 70 Gew.-% p-Hydroxybenzoesäuremethylester und 30 Gew.-% p-Hydroxybenzoesäurepropylesterbesteht, empfohlen (siehe Seite 412, Stichwort Nip-Nip[R]).

Die dauerhafte Konservierung wäßriger alkalischer pharmazeutischer Zubereitungen gestaltet sich problematisch, da die Wirksamkeit der meisten Konservierungsstoffe im alkalischen Milieu nicht mehr uneingeschränkt gegeben ist. Hinsichtlich der Konservierung der genannten Arzneimittelpräparate ist insbesondere eine langanhaltende Konservierung erwünscht, die sich auch über Zeiträume von mehr als zwei Jahre erstreckt.

Es bestand daher die Aufgabe, Konservierungsmittel zur Verfügung zu stellen, die eine gute dauerhafte Konservierung wäßriger alkalischer pharmazeutischer Zubereitungen gewährleisten.

Es wurde nunmehr gefunden, daß wäßrige alkalische pharmazeutische Zubereitungen von Wirkstoffen besonders günstig durch eine konservierend wirksame Menge einer PHB-Esterkombination, welche 50 bis 80 Gew.-% p-Hydroxybenzoesäureethylester und/oder dessen Natriumsalz, 10 bis 25 Gew.-% p-Hydroxybenzoesäurepropylester und/oder dessen Natriumsalz und 10 bis 35 Gew.-% p-Hydroxybenzoesäurebutylester und/oder dessen Natriumsalz enthält, konserviert werden können. Die PHB-Esterkombination erfüllt die an die Konservierung der genannten Arzneimittelpräparate (pharmazeutischen Zubereitungen) gestellten Anforderungen überraschend gut und zeichnet sich durch eine gute Langzeitkonservierung aus. In den erfindungsgemäß konservierten pharmazeutischen Zubereitungen liegen somit der p-Hydroxybenzoesäureethylester, der p-Hydroxybenzoesäurepropylester und der p-Hydroxybenzoesäurebutylester nebeneinander in einer vorteilhaften Kombination mit synergistischer Wirksamkeit vor. Auch kann in den erfindungsgemäß konservierten Zubereitungen jeder der drei genannten Ester durch sein Natriumsalz, welches mit der zur Salzbildung befähigten phenolischen p-Hydroxygruppe der Ester gebildet werden kann, teilweise oder vollständig ersetzt sein.

Die in den erfindungsgemäß konservierten pharmazeutischen Zubereitungen enthaltenen PHB-Esterkombinationen gewährleisten auch schon in sehr geringen Mengen eine gute Konservierung. So reichen hierfür bereits Mengen ab 0,05 Gew.-%, insbesondere von 0,05 Gew.-% bis 0,25 Gew.-% der vorstehend genannten PHB-Esterkombination, bezogen auf die gesamte konservierte pharmazeutische Zubereitung. Um eine Langzeitkonservierung über einen Zeitraum von mehr als zwei Jahren, insbesondere über einen Zeitraum von etwa 3 bis 4 Jahren, sicherzustellen, eignen sich Mengen von 0,1 Gew.-% bis 0,25 Gew.-% der erfindungsgemäß eingesetzten Parahydroxybenzoesäureesterkombination, bezogen auf die gesamte konservierte Zubereitung. Hierbei überrascht umsomehr, daß die gute, langanhaltende Konservierung der wäßrig alkalischen pharmazeutischen Zubereitungen trotz der niedrigen anzuwendenden Mengen der PHB-Esterkombination im vorliegenden wäßrig-alkalischen Milieu der Zubereitungen gewährleistet ist. Die in den erfindungsgemäß konservierten Zubereitungen enthaltenen Konservierungsmittelkombinationen zeigen hierbei auch unter den in den Zubereitungen vorliegenden alkalischen Bedingungen eine gute Hydrolysestabilität, so daß auch bei längerer Lagerung oder Gebrauchsdauer eine Qualitätsverminderung oder gar ein Nachlassen der konservierenden Wirkung bis hin zur völligen Unbrauchbarkeit der Zubereitungen nicht zu befürchten ist.

Die erfindungsgemäß konservierten Zubereitungen können an sich beliebige wäßrige alkalische pharmazeutische Zubereitungen von Wirkstoffen sein. Solche Zubereitungen können z.B. an sich basisch reagierende Wirkstoffe, z.B. Amingruppen-haltige Wirkstoffe oder alkalisch reagierende Salze von schwach sauren Wirkstoffen sein. Beispiele für solche Zubereitungen sind perorale Lösungen und Suspensionen, beispielsweise Antirheumatika wie z.B. Diclofenac-Natrium, Analgetika wie z.B. Paracetamol, Antihistaminika wie z.B. Doxylamin, oder psychotrope Wirkstoffe wie beispielsweise Butyrophenone, z.B. Haloperidol. Auch topisch anwendbare wäßrige Systeme, z.B. Suspensionen, Lösungen oder Emulsionen, beispielsweise Antirheumatika wie Diclofenac-Natrium, Ketoprofen, Etofenamat, oder topisch zu verabreichende Hormone, z.B. β-Estradiol oder Progesteron, sind möglich.

Zweckmäßigerweise besitzen die erfindungsgemäß konservierten pharmazeutischen Zubereitungen einen pH-Wert von unter 9, vorzugsweise einen pH-Wert von 7,5 bis 8,5. In einer vorteilhaften Ausgestaltung der Erfindung sind die konservierten pharmazeutischen Zubereitungen peroral applizierbare Zubereitungen, insbesondere von antaciden Wirkstoffen. Als antacide Wirkstoffe können besonders basische Nitrate, Carbonate, Hydrogencarbonate, Silikate, Silikathydrate, Oxide und/oder Hydroxide von physiologisch verträglichen Metallen oder Gemische derselben in den erfindungsgemäß konservierten Zubereitungen enthalten sein. Beispiele hierfür sind basisches Wismutnitrat, Natriumhydrogencarbonat, Calciumcarbonat, Magnesiumcarbonat, Magnesiumoxid, Magnesiumhydroxid, Aluminiumhydroxid, Magnesium-carbonat-hydroxid, Hydrotalcit (= Aluminium-Magnesium-hydroxid-carbonat-hydrat), Magaldrat (= Magnesium-Aluminat-hydrat). Beispiele für Silikate sind Magnesium-Aluminium-Silikate wie z.B. Magnesiumtrisilikat und Dimagnesium-Aluminiumtrisilikat. Als Silikathydrat kann beispielsweise Magnesium-Aluminiumsilikathydrat in den erfindungsgemäß konservierten Zubereitungen eingesetzt werden. Bevorzugte Ausgestaltungen der erfindungsgemäß konservierten peroral applizierbaren Zubereitungen sind wäßrige Suspensionen der antaciden Wirkstoffe, z.B. von Calciumcarbonat, Aluminiumhydroxid, Magnesiumhydroxid, Aluminium-Magnesiumsilikathydrat, basischen Wismutnitrat, Hydrotalcit oder Magaldrat. Besonders bevorzugte antacide Wirkstoffe sind kolloidal suspendierbare Hydroxide von physiologisch verträglichen Metallen, insbesondere die Wirkstoffe Aluminiumhydroxid (Aluminiumoxid-Gel) und/oder Magnesiumhydroxid.

Die erfindungsgemäß konservierten pharmazeutischen Zubereitungen können in der Weise zubereitet werden, daß man die PHB-Esterkombination gegebenenfalls unter Rühren den pharmazeutischen Zubereitungen zusetzt. So können die Konservierungsmittel entweder nachträglich in den angegebenen Mengen in die pharmazeutischen Zubereitungen z.B. durch Erwärmung gelöst werden oder sie werden bereits in einer zur Herstellung der pharmazeutischen Zubereitung dienenden Wasserphase, ebenfalls meist durch Erwärmung, gelöst. Es können gegebenenfalls zunächst auch Vorlösungen der Konservierungsmittel in Alkohol oder Propylenglycol hergestellt werden, wobei dann diese Vorlösungen den pharmazeutischen Zubereitungen zugesetzt werden.

Die erfindungsgemäß konservierten Zubereitungen können außer dem Wirkstoff und der PHB-Esterkombination noch weitere pharmazeutisch übliche Formulierungs- und Hilfsstoffe enthalten. Beispielsweise werden für Suspensions-Zubereitungen Quellstoffe, Netzmittel, Peptisatoren, und Schutzkolloide als Formulierungsstoffe verwendet. Den oral applizierbaren Zubereitungen können an sich übliche Geschmackskorrigentien wie Süßungsmittel oder Aromastoffe zugesetzt sein. Als Süßungsmittel können insbesondere physiologisch verträgliche Süßstoffe und Zuckeraustauschstoffe oder deren Gemische, wie sie allgemein in der Arzneimittel-, Getränke- und Lebensmittelherstellung Verwendung finden, dienen. Als Aromastoffe können sowohl natürliche, als auch naturidentische oder synthetische Aromastoffe Verwendung finden. Die Formulierungs- und Hilfsstoffe und gegebenenfalls die Geschmackskorrigentien werden in an sich üblichen Mengen für wäßrige Arzneimittelzubereitungen eingesetzt.

Zur Konfektionierung können die erfindungsgemäß konservierten pharmazeutischen Zubereitungen in Mehrfachdosisbehältern, die zur wiederholten Entnahme mit geeigneten Dosierhilfen wie z.B. Dosierlöffeln, Dosierspritzen, Schraubpipetten, Zentraltropfern, Meßbechern oder Dosierventilen (in Kombination mit Druckgasbehältern) ausgestattet sind, als Fertigarzneimittel, beispielsweise in Polypropylen-Flaschen, Hochdruckpolyethylen(HDPE)-Flaschen, Glas-Flaschen oder Aluminiumbehältern mit Innenschutzlackierung abgefüllt werden.

Die nachfolgenden Beispiele sollen die Erfindung weiter erläutern, ohne sie jedoch in ihrem Umfang zu beschränken.


Beispiel I:


Konservierte antacide Suspensionen


Zusammensetzung:

Antacide Wirkstoffe

    Aluminiumoxid (als Aluminiumhydroxidgel) und      900 mg

    Magnesiumoxid (als Magnesiumhydroxidgel)      600 mg

Magnesiumaluminiumsilikat (Quellstoff als      100 mg

viskositätserhöhende Komponente)

Sorbitollösung (70 Gew.-%; Süßungsmittel).     1500 mg

Pfefferminzaroma      4 mg

Konservierungsmittel

    Ethyl-4-hydroxybenzoat      15,0 mg

    Propyl-4-hydroxybenzoat      3,0 mg

    Butyl-4-hydroxybenzoat      5,0 mg

keimfreies dest. Wasser      ad 10 ml

Die Konservierungsmittel wurden in einem Teil des destillierten, keimfreien Wassers bei 65 °C gelöst und mit diesem konservierten Wasser die Quellung mit dem Quellstoff angesetzt. Nach vollständiger Quellung wurde in das gebildete Gel das Süßungsmittel eingerührt und diese Mischung anschließend mit dem Aluminiumhydroxidgel und dem Magnesiumhydroxidgel vereinigt. Bevor mit Wasser auf das Sollvolumen aufgefüllt wurde, wurde noch das Aroma zugesetzt. Abschließend wurde die Mischung nochmals homogenisiert. Man erhielt eine antacide Suspensison der oben angegebenen Zusammensetzung (Zubereitung Nr. 1), die je 10 ml Suspension die Bestandteile in den angegebenen Mengen enthielt.

Analog der antaciden Zubereitung Nr. 1 wurden weitere erfindungsgemäß konservierte Zubereitungen hergestellt. Bei diesen Zubereitungen wurde gegenüber Zubereitung Nr. 1 nur der Gehalt der Konservierungsmittel variiert, während alle anderen Komponenten in einer Menge wie bei Zubereitung Nr. 1 vorlagen. In Tabelle I wird die Variation der Konservierungsmittel angegeben.

Tabelle I

| Menge der Konservierungsmittel in den erfindungsgemäß konservierten Zubereitungen Nr. 2 bis 9 gemäß Beispiel I | | | | | | | | |
|---|---|---|---|---|---|---|---|---|
| Zubereitung Nr. | 2 | 3 | 4 | 5 | 6 | 7 | 8 | 9 |
| Ethyl-4-hydroxybenzoat [mg] | 12,82 | 12,65 | 6,24 | 5,81 | 3,72 | 2,34 | 1,96 | 1,32 |
| Propyl-4-hydroxybenzoat [mg] | 2,56 | 2,56 | 1,54 | 0,85 | 0,79 | 0,78 | 0,40 | 0,35 |
| Butyl-4-hydroxybenzoat [mg] | 4,27 | 4,27 | 3,25 | 0,81 | 1,24 | 0,86 | 0,67 | 0,79 |
| Summe [mg] | 19,65 | 19,48 | 11,03 | 7,47 | 5,75 | 3,98 | 3,03 | 2,46 |

Vergleichsbeispiel

Zu Vergleichszwecken wurde zusätzlich analog den Zubereitungen des Beispiel I folgende Vergleichszubereitung hergestellt, bei der statt der erfindungsgemäßen PHB-Esterkombination ein herkömmliches 2-Komponentengemisch aus Methyl-4-hydroxybenzoat und Propyl-4-hydroxybenzoat nach dem bisherigen Stand der Technik eingesetzt wurde:

| Vergleichszubereitung A: | |
| --- | --- |
| Propyl-4-hydroxybenzoat | 4,27 mg |
| Methyl-4-hydroxybenzoat | 17,09 mg. |

Alle anderen Bestandteile waren identisch mit den Zubereitungen des Beispiel I.

Beispiel II:

Prüfung auf ausreichende Konservierung nach der Methode des DAB 9, Anhang VIII. N1

Die erfindungsgemäß konservierten Zubereitungen nach Beispiel I wurden auf konservierende Wirkung überprüft, indem sie mit den nachfolgend aufgeführten Referenz-Mikroorganismen, die dem Fachmann allgemein als Kontaminationskeime zur Verwendung in Konservierungstests bekannt sind, beimpft wurden. Die Zubereitungen wurden hierfür jeweils so mit einer solchen Menge die Referenz-Mikroorganismen enthaltenden Suspension versetzt, daß eine Keimdichte von $10^5$ bis $10^6$ Mikroorganismen je ml der Zubereitungen entstand.

| Bakterien: | Hefen und Pilze: |
| --- | --- |
| S. Aureus | A. Niger |
| E. Coli | C. Albicans |
| P. Aeruginosa | |

Von den aufgeführten Arten wurden Reinkulturen*verwendet, die bei der "American Type Culture Collection" (ATCC) kommerziell erhältlich sind.

Das zur Beimpfung benutzte Volumen der die Testorganismen enthaltenden Suspension (Inokulum) betrug maximal 1 % des Volumens der Zubereitung. Die beimpften Zubereitungen wurden bei 25 ± 1 °C bebrütet. Aus jeder beimpften Zubereitung wurden jeweils nach 24 Stunden sowie 7, 14, 21 und 28 Tagen Proben entnommen, um die Zahl der koloniebilden den Einheiten (KBE/ml) mit Hilfe von Verdünnungsreihen zu bestimmen. Hierfür wurde eine Reihe von 12 Röhrchen mit je 9 bis 10 ml flüssigem Medium A**bereitgestellt. In jedes der drei ersten Röhrchen wurde 1 ml der im Verhältnis 1:10 verdünnten Zubereitung zugesetzt. In jedes der drei folgenden Röhrchen wurde 1 ml der im Verhältnis 1:100 verdünnten Zubereitung zugesetzt. In jedes der weiterfolgenden drei Röhrchen wurde 1 ml der im Verhältnis 1:1000 verdünnten Zubereitung zugesetzt. In jedes der drei letzten Röhrchen wurde 1 ml der Verdünnungsflüssigkeit zugesetzt. Alle Röhrchen wurden bei 30 bis 35 °C 5 Tage lang bebrütet. Die letzten drei Röhrchen durften kein Wachstum von Mikroorganismen zeigen. Die wahrscheinliche Zahl der Mikroorganismen je ml der Zubereitung wurde anhand der Zahl der Röhrchen, in denen mikrobielles Wachstum, bezogen auf die Menge der zugesetzten Zubereitung, beobachtet wurde, nach einer hierfür erstellten Referenz-Tabelle (siehe z.B. deutsches Arzneibuch, Seite 44, 9. Ausgabe 1986, V.2.1.8) bestimmt.

Für die erfindungsgemäß konservierten Zubereitungen Nr. 2 - 5 wurde die Zahl der koloniebildenden Einheiten (KBE/ml) nach Teststämmen getrennt ermittelt. Tabelle II.1 zeigt das Ergebnis der Keimzahlbestimmung der Bakterien, Tabelle II.2 das Ergebnis der Keimzahlbestimmung der Pilze/Hefen nach den in Tabelle II.1 bzw. Tabelle II.2 angegebenen Lagerzeiten.

* Im hier beschriebenen Test wurden exemplarisch die Stämme E. coli ATCC 8739, P. aeruginosa ATCC 9027, S. aureus ATCC 6538, C. albicans ATCC 10231 und A. niger ATCC 16404 eingesetzt.

** Flüssiges Medium A: Caseinpepton (Pankreashydrolysat) 17,0 g); Sojapepton (Papainhydrolysat): 3,0 g; Natriumchlorid: 5,0 g; Kaliummonohydrogenphosphat: 2,5 g; Glucose-Monohydrat: 2,5 g; keimfreies, destilliertes Wasser: 1000 ml

Tabelle II.1

| Keimzahlbestimmung der Bakterien in den erfindungsgemäß konservierten Zubereitungen Nr. 2-5 gemäß Beispiel II | | | | | | | |
|---|---|---|---|---|---|---|---|
| Keimart | Zubereitung Nr. | Inokulum (KBE/ml) | Keimzahl nach Lagerung von | | | | |
| | | | 24 h | 7d | 14d | 21d | 28d |
| E.coli | 2 | 220.000 | 0 | 0 | 0 | 0 | 0 |
| | 3 | 150.000 | 0 | 0 | 0 | 0 | 0 |
| | 4 | 150.000 | 0 | 0 | 0 | 0 | 0 |
| | 5 | 220.000 | 0 | 0 | 0 | 0 | 0 |
| P.aeroginosa | 2 | 170.000 | 0 | 0 | 0 | 0 | 0 |
| | 3 | 320.000 | <270 | 0 | 0 | 0 | 0 |
| | 4 | 320.000 | 0 | 0 | 0 | 0 | 0 |
| | 5 | 160.000 | <270 | <100 | 0 | 0 | 0 |
| S.aureus | 2 | 300.000 | 0 | 0 | 0 | 0 | 0 |
| | 3 | 150.000 | 270 | 110 | 0 | 0 | 0 |
| | 4 | 150.000 | 810 | 110 | 0 | 0 | 0 |
| | 5 | 350.000 | <270 | 0 | 0 | 0 | 0 |
| KBE = Koloniebildende Einheiten<br>h = Stunden<br>d = Tage | | | | | | | |

Tabelle II.2

| Keimzahlbestimmung der Pilze/Hefen in den erfindungsgemäß konservierten Zubereitungen Nr. 2-5 gemäß Beispiel II | | | | | | | |
|---|---|---|---|---|---|---|---|
| Keimart | Zubereitung Nr. | Inokulum (KBE/ml) | Keimzahl nach Lagerung von | | | | |
| | | | 24 h | 7d | 14d | 21d | 28d |
| C.albicans | 2 | 225.000 | <270 | 0 | 0 | 0 | 0 |
| | 3 | 280.000 | <270 | 0 | 0 | 0 | 0 |
| | 4 | 280.000 | <270 | 0 | 0 | 0 | 0 |
| | 5 | 370.000 | <270 | <100 | 0 | 0 | 0 |
| A.niger | 2 | 240.000 | 0 | 0 | 0 | 0 | 0 |
| | 3 | 320.000 | 0 | 0 | 0 | 0 | 0 |
| | 4 | 320.000 | 0 | 0 | 0 | 0 | 0 |
| | 5 | 310.000 | 190.000 | <100 | 0 | 0 | 0 |
| h = Stunden;<br>d = Tage<br>KBE = Koloniebildende Einheiten | | | | | | | |

Die vorstehenden Keimzahlbestimmungen zeigen, daß sowohl gegenüber Bakterien als auch gegenüber Pilzen/Hefen die erfindungsgemäß konservierten Zubereitungen eine ausgezeichnete konservierende Wirkung aufweisen. Insbesondere zeigen sie bereits nach kurzen Zeiträumen, beispielsweise 24 h, eine sehr gute Keimzahlverminderung. Demgegenüber zeigen nicht erfindungsgemäß konservierten Zubereitungen bei Bakterien eine wesentlich schlechtere Keimzahlverminderung.

In einem Vergleichstest wurde Vergleichszubereitung A mit einem Bakterien-Inokulum, bestehend aus den Keimen E. coli, P. aeruginosa und S. aureus, (KBE/ml = 210.000) beimpft und die Keimzahl nach den

EP 0 523 376 A1

gleichen Zeitabständen wie bei den erfindungsgemäß konservierten Zubereitungen bestimmt. Nach 24 h weist Vergleichszubereitung A eine Keimzahl von 65.000 KBE/ml auf.

Beispiel III:

Ermittlung der minimalen Hemmkonzentration (MHK)

In den konservierten Zubereitungen Nr. 6 bis 9 wurde die minimale Hemmkonzentration (MHK) der erfindungsgemäß eingesetzten PHB-Esterkombination ermittelt. Als minimale Hemmkonzentration wird die kleinste Konzentration bezeichnet, bei der die Vermehrung der Keime gehemmt wird.

Die minimale Hemmkonzentration (MHK) gegenüber bakteriellen Kontaminationen wurde ermittelt, indem die Zubereitungen Nr. 6 bis 9 mit einem Inokulum, bestehend aus den Keimen E. coli, P. aeruginosa und S. aureus, beimpft wurden. Das Inokulum betrug 750.000 KBE/ml (E. coli 160.000 KBE/ml; P. aeruginosa 230.000 KBE/ml; S. aureus 360.000 KBE/ml). Die Keimzahlbestimmung erfolgte wie in Beispiel II beschrieben.

Für die erfindungsgemäße PHB-Esterkombination liegt die minimale Hemmkonzentration (MHK), bei der die verwendeten Bakterienteststämme noch eliminiert werden, bei 246 ppm (entspricht Zubereitung Nr. 9).

Die minimale Hemmkonzentration (MHK) der erfindungsgemäß eingesetzten PHB-Esterkombination für Pilze/Hefen wurde nach Beimpfung mit einem Inokulum, bestehend aus C. Albicans und A. niger, ermittelt. Das Inokulum betrug 240.000 KBE/ml. Die Keimzahlbestimmung erfolgte wie in Beispiel II beschrieben.

In den Zubereitungen Nr. 6-9 war C.albicans nach 7 Tagen nur noch qualitativ nachweisbar (< 100 KBE/ml). Für C.albicans ergibt sich somit ein MHK-Wert für die erfindungsgemäße PHB-Esterkombination von 246 ppm (entspricht Zubereitung Nr. 9). Für A. niger erfolgt eine völlige Wachstumshemmung durch die erfindungsgemäße PHB-Esterkombination bei einer minimalen Hemmkonzentration von 575 ppm (entspricht Zubereitung Nr. 6).

Die Ergebnisse zeigen, daß die in den erfindungsgemäß konservierten Zubereitungen enthaltenen PHB-Esterkombinationen als Konservierungsmittel unerwartet niedrige minimale Hemmkonzentrationen aufweisen.

Beispiel IV:

Aufbrauchtest

Der Aufbrauchtest wurde an drei Mustern durchgeführt, bei denen jeweils 250 ml der erfindungsgemäß konservierten Zubereitung Nr. 1 des Beispiels I in einer ca. 270 ml fassende sterilen Polypropylen-Flasche gelagert wurden. Täglich wurden den Flaschen nach kräftigem Schütteln ca. 1 ml Volumen mit Hilfe eines Dosierlöffels entnommen. Nach 4 Wochen wurden die Proben auf die Anwesenheit folgender Keime überprüft:
- S.aureus
- E.coli
- P.aeruoginosa
- Salmonellen
- Enterobakterien.
Es waren keine Keime in den Lösungen nachzuweisen.

Beispiel V:

Langzeitstabilität der erfindungsgemäßen PHB-Esterkombinationen in alkalischen pharmazeutischen Zubereitungen

Analog der antaciden Zubereitung Nr. 1 wurden drei erfindungsgemäß konservierte alkalische Zubereitungen mit den Bezeichnungen A, B und C hergestellt, in denen anschließend die Langzeitstabilität der erfindungsgemäßen PHB-Esterkombinationen überprüft wurde.

In Tabelle V.1 wird die Menge der PHB-Ester in den Zubereitungen A, B und C angegeben.

7

Tabelle V.1

| Menge der Konservierungsmittel in den erfindungsgemäß konservierten Zubereitungen A, B C gemäß Beispiel I | | | |
|---|---|---|---|
| Zubereitung | A | B | C |
| Ethyl-4-hydroxybenzoat [mg] | 14,4 | 14,3 | 14,4 |
| Propyl-4-hydroxybenzoat [mg] | 3,0 | 2,9 | 3,0 |
| Butyl-4-hydroxybenzoat [mg] | 4,9 | 4,6 | 4,9 |
| Summe [mg] | 22,3 | 21,8 | 22,3 |

Es wurden je ein Muster der Zubereitungen A, B und C steril in Polypropylen-Flaschen abgefüllt und für eine Lagerzeit von 18 Monaten bei einer konstanten Temperatur von 21 °C bzw. von 31 °C gelagert. Zu Beginn der Lagerung sowie nach Ablauf von 12 und 18 Monaten wurden jeweils die Konzentrationen der nichthydrolysierten PHB-Ester in den Mustern ermittelt. Die Bestimmung der PHB-Esterkonzentrationen erfolgte densitometrisch nach dünnschichtchromatographischer Auftrennung der PHB-Estergemische durch Messung der Extinktion der nichthydrolysierten PHB-Ester bei einer Wellenlänge von 254 nm. In allen Mustern lag der pH-Wert während der Lagerung im Bereich von 7,8 bis 7,9.

Tabelle V.2 zeigt die Konzentrationen, wie sie für die einzelnen PHB-Ester in den erfindungsgemäß konservierten Zubereitungen nach Lagerung von 12 und 18 Monaten bei 21 °C ermittelt wurden.

Tabelle V.2

| Ermittelte Konzentrationswerte der PHB-Ester in den erfindungsgemäß konservierten Zubereitungen nach Lagerung von 12 und 18 Monaten bei 21 °C | | | | |
|---|---|---|---|---|
| | Zubereitung | AnfangsKonzentration | Konzentration nach Lagerung für | |
| | | | 12 Monate | 18 M nate |
| Ethyl-4-hydroxybenzoat [mg] | A | 14,4 | 12,2 | 11,7 |
| | B | 14,3 | 12,4 | 11,5 |
| | C | 14,4 | 13,0 | 11,3 |
| Propyl-4-hydroxybenzoat [mg] | A | 3,0 | 2,3 | 2,6 |
| | B | 2,9 | 2,5 | 2,0 |
| | C | 3,0 | 2,5 | 2,5 |
| Butyl-4-hydroxybenzoat [mg] | A | 4,9 | 3,6 | 4,3 |
| | B | 4,6 | 3,9 | 4,5 |
| | C | 4,9 | 3,8 | 4,2 |

Tabelle V.3 zeigt die Konzentrationen, wie sie für die einzelnen PHB-Ester in den erfindungsgemäß konservierten Zubereitungen nach Lagerung von 12 und 18 Monaten bei 31 °C ermittelt wurden.

Tabelle V.3

| Ermittelte Konzentrationswerte der PHB-Ester in den erfindungsgemäß konservierten Zubereitungen nach Lagerung von 12 und 18 Monaten bei 31 °C | | | | |
|---|---|---|---|---|
| | Zubereitung | Anfangskonzentration | Konzentration und Lagerung für | |
| | | | 12 Monate | 18 Monate |
| Ethyl-4-hydroxybenzoat [mg] | A | 14,4 | 8,7 | 6,7 |
| | B | 14,3 | 8,0 | 6,3 |
| | C | 14,4 | 8,4 | 6,8 |
| Propyl-4-hydroxybenzoat [mg] | A | 3,0 | 2,3 | 1,7 |
| | B | 2,9 | 2,4 | 1,6 |
| | C | 3,0 | 2,0 | 1,7 |
| Butyl-4-hydroxybenzoat [mg] | A | 4,9 | 3,4 | 3,2 |
| | B | 4,6 | 2,8 | 3,3 |
| | C | 4,9 | 3,2 | 3,0 |

Die Ergebnisse zeigen, daß die erfindungsgemäß konservierten Zubereitungen eine sehr hohe Langzeitstabilität aufweisen. So liegen auch nach Lagerung von 18 Monaten die Konzentrationen der PHB-Esterkombinationen in allen Mustern deutliche oberhalb der für die erfindungsgemäßen PHB-Esterkombinationen ermittelten minimalen Hemmkonzentrationen.

Mit einem Muster der Zubereitung A wurde zusätzlich noch eine Prüfung auf ausreichende Konservierung durchgeführt. Hierfür wurde nach einer Lagerung von 12 Monaten bei einer konstanten Temperatur von 21 °C das Muster, wie in Beispiel II beschrieben, mit einem Inokulum von $10^5$ bis $10^6$ KBE/ml beimpft und nach 24 Stunden sowie 7 Tagen die Keimzahl im Muster ermittelt. Nach Ablauf von 24 Stunden und nach Ablauf von 7 Tagen war in der Zubereitung nurmehr A. Niger qualitativ nachweisbar (< 100 KBE/ml). Damit wird deutlich, daß erfindungsgemäß konservierte Zubereitungen auch nach Langzeitlagerung noch eine ausgezeichnete konservierende Wirkung zeigen.

**Patentansprüche**

1. Konservierte wäßrige alkalische pharmazeutische Zubereitungen von Wirkstoffen, dadurch gekennzeichnet, daß sie als Konservierungsmittel eine Parahydroxybenzoesäureesterkombination aus 50 bis 80 Gew.-% p-Hydroxybenzoesäureethylester und/oder dessen Natriumsalz, 10 bis 25 Gew.-% p-Hydroxybenzoesäurepropylester und/oder dessen Natriumsalz und 10 bis 35 Gew.-% p-Hydroxybenzoesäurebutylester und/oder dessen Natriumsalz enthalten.

2. Pharmazeutische Zubereitungen nach Anspruch 1, dadurch gekennzeichnet, daß sie die Parahydroxybenzoesäureesterkombination in einer Menge von 0,05 bis 0,25 Gew.-%, bezogen auf die gesamte pharmazeutische Zubereitung, enthalten.

3. Pharmazeutische Zubereitungen nach Anspruch 2, dadurch gekennzeichnet, daß sie die Parahydroxybenzoesäureesterkombination in einer Menge von 0,10 bis 0,25 Gew.-%, bezogen auf die gesamte pharmazeutische Zubereitung, enthalten.

4. Pharmazeutische Zubereitungen nach einem der vorhergehenden Ansprüche, dadurch gekennzeichnet, daß sie einen pH-Wert von unter 9, vorzugsweise einen pH-Wert von 7,5 bis 8,5, besitzen.

5. Pharmazeutische Zubereitungen nach einem der vorhergehenden Ansprüche, dadurch gekennzeichnet, daß sie peroral applizierbare Zubereitungen sind.

6. Pharmazeutische Zubereitungen nach Anspruch 5, dadurch gekennzeichnet, daß sie antacide Wirkstoffe enthalten.

7.  Pharmazeutische Zubereitungen nach Anspruch 6, dadurch gekennzeichnet, daß die antaciden Wirkstoffe basische Nitrate, Carbonate, Hydrogencarbonate, Silikate, Silikathydrate, Oxide und/oder Hydroxide von physiologisch verträglichen Metallen sind.

8.  Pharmazeutische Zubereitungen nach Anspruch 7, dadurch gekennzeichnet, daß die antaciden Wirkstoffe Aluminiumhydroxid und/oder Magnesiumhydroxid sind.

9.  Verfahren zur Herstellung konservierter wäßriger alkalischer pharmazeutischer Zubereitungen von Wirkstoffen, dadurch gekennzeichnet, daß man als Konservierungsmittel eine Parahydroxybenzoesäureesterkombination aus 50 bis 80 Gew.-% p-Hydroxybenzoesäureethylester und/oder dessen Natriumsalz, 10 bis 25 Gew.-% p-Hydroxybenzoesäurepropylester und/oder dessen Natriumsalz und 10 bis 35 Gew.-% p-Hydroxybenzoesäurebutylester und/oder dessen Natriumsalz den pharmazeutischen Zubereitungen zusetzt.

Europäisches
Patentamt

**EUROPÄISCHER RECHERCHENBERICHT**

Nummer der Anmeldung

EP    92 10 9977
Seite 1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | JOURNAL OF PHARMACEUTICAL SCIENCES Bd. 73, Nr. 1, Januar 1984, Seiten 128 - 131 SCHIEFFER G.W. ET AL 'SIMULTANEOUS DETERMINATION OF METHYL,ETHYL ,PROPYL,AND BUTYL 4-HYDROXYBENZOATES AND 4-HYDROXYBENZOIC ACID IN LIQUID ANTACID FORMULATIONS BY GAS CHROMATOGRAPHY' | 1,5,6,9 | A61K47/14 |
| Y | * Seite 128, Absatz 1 - Seite 129, Absatz 1 * *seite 130, "Sample and standard solution"* --- | 2-4,7,8 | |
| Y | WO-A-8 404 886 (SUTTON LABORATORIES INC.) 20. Dezember 1984 * Seite 7 - Seite 8; Beispiel v * * Ansprüche 1,2,5 * --- | 2,3,7,8 | |
| Y | GB-A-2 086 760 (MALLINKRODT INC) 23. Oktober 1980 * Seite 2, Zeile 40 - Zeile 51 * * Seite 2; Beispiel 1 * --- | 4 | |

**RECHERCHIERTE SACHGEBIETE (Int. Cl.5)**

| Kategorie | Kennzeichnung des Dokuments | Betrifft Anspruch | |
|---|---|---|---|
| Y | ROTH H.J. ET AL 'hagers handbuch der pharmazeutischen praxis' 1977 , SPRINGER VERLAG , BERLIN-HEIDELBERG-NEW YORK * Seite 312 * * Seite 321 * --- | 4 | A61K A23L A01N |

-/--

Der vorliegende Recherchenbericht wurde für alle Patentansprüche erstellt

| Recherchenort | Abschlußdatum der Recherche | Prüfer |
|---|---|---|
| DEN HAAG | 27 OKTOBER 1992 | BOULOIS D. |

<table>
<tr><td>Europäisches<br>Patentamt</td><td>EUROPÄISCHER RECHERCHENBERICHT</td><td>Nummer der Anmeldung<br><br>EP 92 10 9977<br>Seite 2</td></tr>
</table>

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | JOURNAL OF PHARMACEUTICAL SCIENCES Bd. 72, Nr. 2, Februar 1983, Seiten 196 - 198 CARNEVALE L. 'SIMULTANEOUS DETERMINATION OF ACETAMINOPHEN, GUAIFENESIN, PSEUDOEPHEDRINE, PHOLCODINE, AND PARABEN PRESERVATIVES IN COUGH MIXTURES BY HIGH-PERFORMANCE LIQUID CHROMATOGRAPHY' *seite 196 "Abstract"* *seiten 196-197, "Standard Solutions"* --- | 1 | |
| A | EP-A-0 072 662 (TAKEDA CHEMICAL INDUSTRIES LTD) 23. Februar 1983 * Seite 3, Zeile 11 - Zeile 17 * * Seite 3, Zeile 26 - Seite 4, Zeile 14 * ----- | 1 | |
| | | | RECHERCHIERTE SACHGEBIETE (Int. Cl.5) |

Der vorliegende Recherchenbericht wurde für alle Patentansprüche erstellt

| Recherchenort | Abschlußdatum der Recherche | Prüfer |
|---|---|---|
| DEN HAAG | 27 OKTOBER 1992 | BOULOIS D. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
......................................................................................
& : Mitglied der gleichen Patentfamilie, übereinstimmendes Dokument

EPO FORM 1503 03.82 (P0403)